# EUROPEAN PATENT SPECIFICATION

(11) **EP 2 932 973 B1**
(45) Date of publication and mention of the grant of the patent: **01.04.2020**
(21) Application number: 15167184.9
(22) Date of filing: 20.04.2011
(51) Int. Cl.: A61K 31/74, A61P 17/00, A61P 17/06, A61K 41/00

(54) **FORMULATIONS FOR USE IN THE TREATMENT OF SKIN CONDITIONS**
FORMULIERUNGEN ZUR VERWENDUNG BEI DER BEHANDLUNG VON HAUTKRANKHEITEN
FORMULATIONS POUR UNE UTILISATION DANS LE TRAITEMENT D'AFFECTIONS DE LA PEAU

(30) Priority: 04.05.2010 US 331131 P
(43) Date of publication of application: 21.10.2015
(62) Divisional of application: 11777830.8
(73) Proprietor: Soligenix, Inc., Princeton, NJ 08540 (US)
(72) Inventor: Tobia, Alfonso, J., Doylestown, PA Pennsylvania 18901 (US); Cabana, Bernard, E., Biglerville, PA Pennsylvania 17307 (US); Rook, Alain, H., Wynnewood, PA Pennsylvania 19096 (US)
(74) Representative: Winter, Brandl, Fürniss, Hübner, Röss, Kaiser, Polte - Partnerschaft mbB

(56) References cited:
- WO-A1-2009/066294
- US-A- 6 001 882
- Floyd E. Fox ET AL: "Photoactivated Hypericin is an anti-Proliferative Agent that Induces a High Rate of Apoptotic Death of Normal, Transformed, and Malignant T Lymphocytes: Implications for the Treatment of Cutaneous Lymphoproliferative and Inflammatory Disorders", , 1 August 1998 (1998-08-01), XP055080120, Retrieved from the Internet: URL:http://www.nature.com/jid/journal/v111 /n2/pdf/5600130a.pdf [retrieved on 2013-09-19]
- VAN DE PUTTE MARIE ET AL: "The impact of aggregation on the biodistribution of hypericin", INTERNATIONAL JOURNAL OF ONCOLOGY, LYCHNIA, GR, vol. 28, no. 3, 1 March 2006 (2006-03-01), pages 655-660, XP009172750, ISSN: 1019-6439
- ROOK A H ET AL: "A phase II placebo-controlled study of photodynamic therapy with topical hypericin and visible light irradiation in the treatment of cutaneous T-cell lymphoma and psoriasis", JOURNAL OF THE AMERICAN ACADEMY OF DERMATOLOGY, C.V. MOSBY, ST. LOUIS, MO, US, vol. 63, no. 6, 1 December 2010 (2010-12-01), pages 984-990, XP027499049, ISSN: 0190-9622, DOI: 10.1016/J.JAAD.2010.02.039 [retrieved on 2010-11-18]
- SARIGUELLUE OZGUENEY ISIK ET AL: "Transdermal delivery of diclofenac sodium through rat skin from various formulations", AAPS PHARMSCITECH, SPRINGER NEW YORK LLC, US, vol. 7, no. 4, 1 December 2006 (2006-12-01), pages 88/E1-E7, XP009172681, ISSN: 1530-9932

## Description

### FIELD OF THE INVENTION

The present invention relates to liquid formulations for use in treating skin conditions. Particularly, the present invention relates to liquid formulations for use in treating skin conditions using photodynamic therapy. The invention is set out in the claims.

### BACKGROUND OF THE INVENTION

Photodynamic therapy (PDT) is a treatment that uses a compound, generally referred to as a photosensitizer or photosensitizing agent, and a particular type of light. Photosensitizers may be naturally occurring or synthesized. When photosensitizers are exposed to a specific wavelength of light, they exert cytotoxicity effect on nearby cells, which may cause cell death in these cells. The photosensitizers have shown to exhibit remarkable anti-cancer and antiviral activities.

Cutaneous T cell lymphoma (CTCL), which includes mycosis fungoides (MF) and Sezary syndrome, are malignancies of skin-trafficking T-cells and represent the most common types of T cell lymphoma. CTCL affects approximately 25,000 to 50,000 individuals in the United States. MF most commonly presents with limited skin involvement, manifested as scaly, erythematous patches or thin plaques. As MF progresses, patients develop more infiltrated skin lesions (thick plaques or tumors), and eventually lymph node, blood or visceral organ involvement. Histopathologically, early MF skin lesions are characterized by T cell infiltrates in the epidermis and upper dermis.

Psoriasis is a chronic, autoimmune skin disease characterized by T -cell mediated inflammation resulting in increased epidermal cell proliferation. It is typically associated with intense T -cell lymphocytic and neutrophilic infiltrates superficially in lesional skin. Psoriasis affects approximately 2% of the total population in the US. According to the National Institutes of Health, as many as 7.5 million Americans have psoriasis. The sexes are equally affected, and the onset can occur from infancy to old age.

Verrucae (warts) are the most common manifestation of infection with the human papilloma virus (HPV). They can occur on any cutaneous or mucosal surface. The diagnosis of verruca is based primarily on clinical morphology. Lesions can frequently be chronic. Immuno-suppressed individuals are at increased risk for the development of squamous cell carcinoma at sites of HPV infection. Furthermore, genital infection with HPV has been implicated as a frequent cause of squamous cell carcinoma of the cervix and genital region. Warts are not commonly treated, at the present time, by photodynamic therapy.

Combinations of photosensitizing agents and ultraviolet (UV) light are established treatments of CTCL and psoriasis. Psoralen ultraviolet-A light (PUV A) treatment, a commonly used regimen for the treatment of psoriasis, is a form of PDT. PUVA treatment involves the administration to a patient of a psoralen compound, followed by illumination of the skin of the patient with light having a wavelength corresponding to ultraviolet A (UV-A) radiation. Both CTCL and psoriasis exhibit high clinical response rates to PUV A therapy. Ultraviolet A, also known as long wave UV, or black light, typically has wavelength of 315 nm - 400 nm. Although PUV A treatment provides relief from psoriasis, exposure of skin to UV -A radiation can have a number of undesirable effects, including a sunburn-like reaction and induction of skin tumors. Additionally, PUV A appears to exert carcinogenic effects with prolonged treatment resulting in a significant rate of non-melanoma skin cancers as well as malignant melanoma. The finding that psoralen is mutagenic and intercalates into DNA, coupled with the association of PUV A therapy with skin cancer development, raises the need for safer forms of phototherapy in the treatment CTCL and psoriasis.

Hypericin is a natural compound found in stems and petals of plants of the genus *hypericum.* Within this genus are eight families and 43 species, including the common St. John's wort plant, *Hypericum perforatum.* Hypericin is the principle phototoxic agent in St. John's wort. The chemical name is 4,5,7,4',5' ,7' -dimethyl-meso naphthodiantbrone, a compound composed of eight conjugated rings containing six hydroxyl groups, two carbonyl and two methyl groups in a symmetric pattern when inverted about a central axis. When dissolved in ethanol solutions it produces a bright fluorescent compound.

Hypericin is maximally activated by visible light at wavelengths produced by a sodium lamp (590 nm) or cool white fluorescent light which significantly reduces the side effects associated with UV irradiation and permits deeper skin penetration. Hypericin has a relatively long half-life (20-24 hours) which permits repeated light activation with single doses. This compound has been demonstrated in skin after systemic administration and it has been shown to be about 20% bioavailable following oral administration. However, patients who were systemically treated with hypericin may be affected by systematic and severe photosensitivity.

The photodynamic properties of hypericin have been described. Upon visible or UV light irradiation, hypericin in solution is capable of exciting oxygen to its singlet state and generating superoxide radicals, which can lead to oxidation of tryptophan imidazole groups in proteins, to oxidation of fatty acids in biological systems, or to other chemical reactions. Hypericin is maximally activated by light of about 570-650 nm wavelength, i.e. in the yellow region of the electromagnetic spectrum. Although hypericin has been known for many years, and although the application of non-photoactivated hypericin has been investigated in clinical studies of its anti-cancer and antiviral activities, very little is known about the mechanism of action of this compound at the biochemical or molecular level. Photoreaction of hypericin has been demonstrated to produce singlet oxygen. In addition, there is evidence that hypericin inhibits protein kinase activity and thus, may exert its biological activity through pathways which are very different from, for example, psoralen. Furthermore, hypericin binds to phospholipids, such as cell membranes comprising phosphatidylcholine, and to retroviral particles, probably by associating with the lipid envelope thereof.

Fox et al (U.S. 6,001,882) discloses methods of using hypericin and hypericin derivatives which, when photoactivated using visible or UV-A light, are useful for treating numerous diseases and disorders in mammals. The hypericin and hypericin derivatives are used for inhibiting the proliferation and accumulation of mammalian leukocytes and for inducing apoptosis in mammalian leukocytes. Topical applications of hypericin and hypericin derivatives for the treatment of CTCL and psoriasis are also contemplated by Fox et al.

Floyd E. Fox et al. ('URL;http://www.nature.com/jid/journal/v111/n2/pdf/5600130a.pdf) reports on studies on the effect of hypericin in cutaneous T cell lymphoma, in which stock solutions of hypericin and 8-methoxypsoralen in benzyl alcohol were used. Van De Putte Marie et al. (International Journal of Oncology. Lychina, GR.,vol. 28, no. 3, 2006-03-01) reports on studies on the biodistribution of hypericin after injection of hypericin suspended in phosphate-buffered saline, or solution of hypericin in a mixture of DMSO, polyethylene glycol and water. WO 2009/066294 A1 discloses devices for photodynamic therapy, which comprise composition for treatment of skin disorders, e.g. psoriasis. The preferred photosensitizer is hypericin; while gels/creams/ointment may prepared, the carrier can also comprise a solvent selected from water, ethanol, butylene glycol, propylene glycol, isopropyl alcohol, isoprene glycol, glycerin.

Presently, there is a need for novel and highly effective therapeutics formulations for the treatment of skin conditions associated with lymphocyte infiltration, particularly psoriasis, and also methods for using said formulations for the treatment of such skin condition.

### DETAILED DESCRIPTION OF THE INVENTION

Hypericin is a known photodynamic agent with the potential to treat a variety of skin diseases characterized by lymphocytic infiltrates, including psoriasis, lichen planus, and CTCL. Similar to other known photosensitizing agents, singlet oxygen is the principal product in the photosensitization of hypericin. In the presence of light irradiation, hypericin excites oxygen to its singlet state and is capable of generating superoxide radicals which can lead to oxidation of tryptophan imidazole groups in proteins and to oxidation of fatty acids in biological systems. Hypericin is maximally activated by light in the yellow region of the photodynamic spectrum at approximately 570-650 nm wavelength. In addition, hypericin, photoactivated with either UV -A or white light, is capable of near complete apoptosis (94%) of cultured malignant CTCL cells.

In a Phase I clinical trial conducted on healthy subjects, the minimal phototoxic dose (MPD) of visible light of topically administered hypericin in hydrophilic ointment was determined to be 4 J/cm2, whereas at 8 J/cm2 brisk phototoxic reactions were observed. At concentrations of 0.1% and 0.5%, hypericin causes cutaneous photosensitivity when topically applied under occlusion and activated by visible light 24 hours after topical application. There were no cutaneous reactions at 0.02% hypericin at any dose of light.

Based on data obtained in the Phase I trial, which identified concentrations of hypericin and doses of light causing cutaneous photosensitive responses in volunteers, a multi-center, Phase II open-label, placebo-controlled study was conducted to evaluate the safety, tolerance, and efficacy of topical hypericin and visible light in the treatment of early
MF and psoriasis vulgaris. The methods and results of which are detailed in the following study. It is unexpectedly discovered that the conventional topical hypericin formulations, such as hydrophilic ointment, are less than satisfactory in clinical studies for the treatment of psoriasis, whereas liquid formulations of hypericin and derivatives are shown to be highly effective for the treatment of psoriasis.

This study is designed to evaluate the safety and efficacy of topical hypericin in concentrations of 0.1% and 0.25% under occlusion for 24 hours followed by administration of visible light in doses ranging from 8 to 20 J/cm2 twice weekly for 6 weeks for the treatment of psoriasis or MF. One of the four centers involved in the study administered topical hypericin in concentrations of 0.05 and 0.1 %. The concentration of hypericin and the dose of light were based upon the Phase I study described above with normal volunteers.

A standardized topical amount of hypericin in either ointment or liquid formula was applied to defined plaques on the skin of volunteer subjects. The amount of hypericin in each topical application was as follows: 0.05% = 0.005 mg/cm²; 0.1% = 0.01 mg/ cm², and 0.25% = 0.025 mg/ cm². Each treated patient had comparable lesions treated with active drug and placebo preparation.

Each hypericin treatment or placebo was applied twice weekly for a period of 24 hours prior to irradiation. Each application site was covered by non-absorbing Webril cotton of equal area and occluded with occlusive tape (Blenderm, 3M); excess drug was removed prior to irradiation (24 hours post drug application).

Irradiation was undertaken using visible fluorescent bulbs (590-650 nm) (GE cool white fluorescent bulbs, 20-watt fluorescent tubes housed in a reflecting bank) for a period not to exceed 15 minutes (total number of joules approximately 5/ cm²). The light dose was administered twice weekly, separated by at least one day.

The light dose was increased once weekly by 1 joule/ cm² until symptoms or signs of phototoxicity appeared. At that point, the dose could either be maintained or reduced by 1 joule/cm² if phototoxicity was pronounced (skin erythema of greater than 1 + [minimal visible erythema).

Skin reactions were evaluated each week immediately after irradiation and again at 48 and 72 hours later. The sites of irradiation were examined twice per week thereafter and all changes; e.g., erythema, edema, desquamation, pigmentation, etc. were recorded.

Lesion responses in this study were scored in accordance with the following scales at each patient evaluation: lesional CR=complete regression of the treated lesion; PR=>50% decrease in lesional area during treatment; Stable=<50% decrease in size or <25% increase in size during therapy; Progression=>25% increase in size during therapy. The success/failure criterion for each lesion on each patient was whether there was a 50% or better improvement in the lesion area at the end of the 6-wcck treatment cycle. If so, the patient was scored as a responder for that lesion. Table 1 indicates the rating criteria for patient response.

**Table 1. Criteria for Treatment Response**

| **Descriptor MF** | **Criterion** | **Number Score** |
|---|---|---|
| Worse | Increase in lesion size | 7 |
| No Response | No change in lesion size | 6 |
| Poor Response | < 25% reduction in size | 5 |
| Fair Response | > 25% reduction but < 25% | 4 |
| Good Response | >= 50% reduction but < 75% | 3 |
| Excellent Response | >=75% reduction but < 100% | 2 |
| Complete Response | 100% Reduction/Complete Resolution | 1 |
| Psoriasis | "Response" at 6 weeks required that combined lesion scores be 50% or less of baseline lesion scores for scaling, erythema, and plaque elevation. | |

Male or non-pregnant female patients 18 to 70 years of age with stable patch or plaque-phase CTCL or stable plaque type psoriasis vulgaris of at least 4 months were to be entered into this study in the two disease arms of the study (MF and psoriasis) at 4 study centers. Planned patient enrollment was 10- 12 CTCL and 10- 12 psoriasis patients. A total of 12 patients were enrolled for the MF arm (all 12 were evaluable) and 13 patients were enrolled into the psoriasis arm (11 were evaluable).

Chemically synthesized hypericin was supplied by VimRx Phan11aceuticals of Wilmington, Delaware. Hypericin was prepared as a stock solution in propylene glycol with an appropriate amount of hypericin stock added to a melted hydrophilic base containing methyl paraben, propyl paraben, sodium lauryl sulphate, stearyl alcohol, white petrolatum, and purified water (USP Hydrophilic Ointment, a non-limiting example of hydrophilic ointment is an ointment base consisting of 25% each of white petrolatum and stearyl alcohol, 12% propyl glycol emulsified in 37% water by 1% oflauryl sulfate; preserved with paraben.). Placebo ointment was used as a control treatment.

All patients receiving treatment for MF were treated with the ointment preparation (reference). For patients with psoriasis, 5 patients were treated with 0.1% and 0.25% aqueous solutions of hypericin in dimethyl sulfoxide (DMSO) and 6 patients were treated with the USP Hydrophilic Ointment preparations. The liquid dosage form consisted of dissolving either 0.2 mL or 0.5 mL hypericin stock solution (polyethylene glycol solution) in 44.8 or 44.5 mL of 50% DMSO/water and mixing with a magnetic stirrer.

The response or lack of response, of each lesion treated with either placebo ointment or with a hypericin-containing ointment was analyzed as a binomial (i.e., yes/no) distribution. This was an appropriate analysis for response which was quantal in nature. For the null hypothesis (no treatment response) it was assumed that spontaneous resolution of the lesion by the protocol specified 6-week time point would not occur. That is, the true probability of spontaneous lesion regression (defined as < 50% of its baseline size for CTCL and to < 50% of its combined baseline score tor psoriasis lesions) was taken as zero (0%). For the MF analysis, a repeated measures logistic regression was employed to obtain p-values. For analysis of the psoriasis data, a likelihood ratio chi-square test was employed.

The study population in both treatment arms of the study contained approximately twice as many males (n = 8 for MF and n = 7 for psoriasis treatment groups) as females (n =4 for both MF and psoriasis treatment groups); the average age of the patients for MF patients was 55± 16.5 years (mean± SO) and for the psoriasis patients 45 ± 16.4 years, (see Table 2 for patient characteristics). Five patients discontinued prematurely; one subject revoked conditional enrollment and 4 discontinued because the protocol was too demanding. Two of these patients were psoriasis patients who were treated for only one day before discontinuing and were thus not evaluable for efficacy.

**Table 2. Demographic Data for All Evaluable Patients Treated for CTCL or Psoriasis**

| | **Diagnosis** | |
|---|---|---|
| | **MF** | **Psoriasis** |
| Number of Patients | 12 | 11 |
| Age (Mean± SD) | 55± 16.5 | 45 ± 16.5 |
| Number of Males/Females | 8/4 | 7/4 |
| Number Treated> 6 Weeks | 7 | 2 |

### Efficacy Results for MF

A lesion was considered to have responded to treatment if the lesion size was reduced by 50% or more following 6 weeks of treatment with hypericin ointment. Results following 6 weeks of treatment are summarized in Tables 3 and 4.

**Table 3. Individual Patients' MF Lesion Responses to Hypericin Ointment Following Six Weeks of Treatment**

| **Hypericin Treatment** | | | | | |
|---|---|---|---|---|---|
| **Site Patient** | **Placebo** | **0.05%** | **0.1%** | **0.25%** | **Responder?** |
| CW-001 | N | - | Y | Y | Y |
| CW-004 | N | - | N | N | N |
| CW-007 | Y | - | N | N | N |
| CW-010 | N | - | N | Y | Y |
| CW-012 | N | - | N | Y | Y |
| CW-016 | N | - | N | N | N |
| A-001 | N | - | N | N | N |
| A-002 | N | - | Y | Y | Y |
| A-003 | N | - | Y | Y | Y |
| C-001 | N | N | N | - | N |
| C-002 | N | Y | Y | - | Y |
| MDA-001 | N | - | Y | n/a | Y |

The overall trend across the two highest doses was similar for effects of hypericin on reduction of MF lesions 2': 50%; the sample size for the 0.05% dose was insufficient to draw any conclusions about efficacy (Table 4). At hypericin concentrations of 0.1% and 0.25%, 41.7 (5 of 12) and 55.6% (5 of 9) patients responded to treatment; the responses to both of these treatments were significantly different from lesions treated with placebo ointment (p 2: 0.04 for both concentrations). The overall conclusion is that hypericin is effective against MF lesions when applied topically at strengths of 0.1% to 0.25% twice weekly followed by white light irradiation 24 hours later at 8 - 20 J/cm2 of skin.

**Table 4. Summary of MF Lesion Responses to Hypericin Ointment Following Six Weeks of Treatment**

| **Treatment Group** | **Responders/Total^{a}** | **Percent Responders^{a}** |
|---|---|---|
| All Hypericin Responders | 7/12 | 58.3%^{b} |
| Hypericin 0.25% Dose | 5/9^{c} | 55.6%^{b} |
| Hypericin 0.1% Dose | 5/12 | 41.7%^{b} |
| Hypericin 0.05% Dose | 1/2 | 50.0% |
| Placebo Responders | 1/12 | 8.3% |

| | | |
|---|---|---|
| a Data reflects the number of patients who had a response to any dose; some patients may have been responders at more than one dose of treatment. b p <= 0.04 versus placebo (exact binomial limits) c Three patients were not treated with the 0.25% dose of hypericin. | | |

### Efficacy Results for the Treatment of Psoriasis

The distribution of psoriasis patient responses to placebo and hypericin concentrations of 0.05%, 0.1 %, and 0.25% at the protocol specified 6-week evaluation time is provided in Tables 5 and 6. In reviewing Table 6, it is important to keep in mind that the first 5 patients at one of the sites, the University of Pennsylvania, were treated with hypericin ointments (reference) while the 6 remaining psoriasis patients at the other sites were treated with a liquid formulation of hypericin in aqueous DMSO/water. The exceptions to this included the sixth University of Pennsylvania patient who was treated with ointment and MD Anderson patient, MDA-002, who was treated with 0.1% and 0.25% hypericin ointment.

For the two higher strength formulations (0.1% and 0.25%) when data for both ointment and liquid formulations were combined, the pairwise comparison of 0.25% (45.4% responders) and 0.1% treatments (36.4% responders) compared to lesions treated with placebo demonstrated a statistically significant effect (p < 0.02 for both concentrations) of hypericin on psoriasis lesion reduction. The pairwise comparisons for the lowest strength treatment (0.05% for both formulations combined) did not show a significant difference compared to placebo. Results for patients treated with the liquid formulation only were similar to those when data from both formulations were combined (Table 6). The dose trend for the liquid formulation alone demonstrated dose related effects of hypericin on psoriasis lesion reduction/amelioration.

**Table 5. Response of Psoriasis to Hypericin Following Six Weeks of Treatment Hypericin Treatment**

| **Site Patient** | **Placebo** | **0.05%** | **0.1%** | **0.25%** | **Responder?** |
|---|---|---|---|---|---|
| CW-006 | N | - | Y | Y | Y |
| CW-011 | N | - | N | N | N |
| CW-014 | N | - | Y | Y | Y |
| CW-015 | N | - | Y | Y | Y |
| CW-017 | | - | N | Y | Y |
| P-001 | N | N | N | - | N |
| P-003 | N | N | N | - | N |
| P-004 | N | N | N | - | N |
| P-005 | N | N | N | - | N |
| P-006 | N | Y | N | - | Y |
| MDA-002 | N | - | Y | Y | Y |

Efficacy results for the ointment formulation were much lower than those for the liquid treatment at all doses tested with none of the doses of ointment demonstrating a statistically significant difference (p > 0.05) from patients treated with placebo.

Overall, the conclusion is that hypericin in the liquid formulation was highly effective against psoriasis when applied topically at concentrations of 0.1% to 0.25% followed by white light irradiation 24 hours later at 8 20 J/cm2 of skin. The ointment formulation did not produce effects statistically better than placebo at any dose tested.

**Table 6. Summary of Psoriatic Lesion Responses to Hypericin Ointment Following Six Weeks of Treatment**

| **All Ointment + Liquid** | **Responders/Total^{a}** | **Percent Responders^{a}** |
|---|---|---|
| All Hypericin Responders | 6/11 | 54.6%^{b} |
| Hypericin 0.25% Dose | 5/11 | 45.4%^{b} |
| Hypericin 0.1% Dose | 4/11 | 36.4%^{a,b} |
| Response to 0.05% Dose | 1/11 | 9.1% |
| Placebo Responders | 0/11 | 0.00% |
| **Liquid** | | |
| All Hypericin Responders | 4/5 | 80.0%^{b} |
| Hypericin 0.25% Dose | 4/5 | 80.0%^{b} |
| Hypericin 0.1% Dose | 3/5 | 60.0%^{b} |
| **Ointment** (reference) | 0/5 | 0.0% |
| All Hypericin Responders | 2/6 | 33.3% |
| Hypericin 0.25% Dose | 1/6 | 16.7% |
| Hypericin 0.1% Dose | 1/6 | 16.7% |
| Response to 0.05% Dose | 1/6 | 16.7% |
| Placebo Responders | 0/6 | 0/6 |

| | | |
|---|---|---|
| a Data reflects the number of patients who had a response to any dose; some patients may have been responders at more than one dose of treatment. b p <= 0.02 versus placebo (exact binomial limits) | | |

No deaths or serious adverse events were reported during the conduct of this study. The only reported adverse events were mild to moderate burning, itching, erythema, and pruritus at the application site typical of phototoxic reactions expected from the study drug.

Based upon the basic laboratory observations that hypericin with visible light exposure was able to induce a high rate of apoptosis of peripheral blood lymphocytes, both transformed and non-transformed, the hypothesis was generated that hypericin would likely be efficacious in the treatment of a broad variety of lymphocytic disorders of the skin including psoriasis, CTCL, lichen planus, and others. The results of this Phase II study confirm and extend the *in vitro* observations with regard to therapeutic efficacy for both psoriasis and MF.

In the MF arm of the study, only the ointment formulation was tested at concentrations of 0.05%, 0.1% and 0.25% (reference). Patients were treated twice weekly with active and placebo ointments followed by exposure to visible light at 8 to 20 J/cm2 24 hours following application. The two higher concentrations of hypericin ointment (0.1 and 0.25%) produced greater than 50% reductions of CTCL plaques in 41.7 and 55.6% patients, respectively. These changes were statistically significantly greater than those observed for placebo-treated lesions in the same patients. At the lowest hypericin concentration tested, 0.05%, one of two patients treated exhibited at least 50% reductions in CTCL lesions; because of the limited size of this population, no conclusion can be drawn concerning efficacy of this concentration.

For patients with psoriasis lesions, two formulations (liquid or ointment) were employed at different sites in concentrations of 0.05%, 0.1 %, and 0.25%. Reductions in psoriasis lesions of greater than 50% were only observed in one of six (16.7%) patients in response to all three concentrations of hypericin ointment (reference); these results were not statistically different from lesions in the same patients treated with placebo. In contrast, lesions treated with the liquid formulation produced statistically significant reductions in the size of psoriasis lesions at concentrations of 0.1% (60.0% of patients) and 0.25% (80%) compared to lesions treated with placebo in the same patients. The 0.05% concentration of the liquid hypericin formulation was not tested. The basis for the differences in response of psoriasis lesions to the liquid and ointment formulations is unknown but is likely related to a greater extent of penetration of the liquid formulation into the lesions.

Both hypericin formulations were safe with the majority of patients experiencing only mild to moderate phototoxicity at the site of application following exposure to visible light.

The mechanism by which topical photoactivated hypericin provides clinical benefit to treat inflammatory skin diseases characterized by lymphocytic infiltrates includes generation of singlet oxygen and apoptosis, and is a similar mechanism to that of other agents, such as 5-aminolevulinic acid and other porphyrins used in photodynamic therapy which are known to have clinical efficacy in the treatment of inflammatory skin diseases. Typically, apoptosis mediated by reactive oxygen species is executed predominantly via the intrinsic (mitochondrial) pathway. This is particularly advantageous for disorders such as CTCL that have been shown often to exhibit defective extrinsic (death receptor mediated) apoptotic pathways including FAS and TRAIL. Furthermore, studies on mechanism of anti-tumor activity indicate that hypericin binds to the chaperone protein, heat shock protein 90 (Hsp 90) leading to its ubiquitinylation. This disrupts several critical client proteins that regulate cell growth pathways resulting in their destabilization, rapid degradation, and elimination ultimately leading to cell death. Moreover, hypericin is known to be a potent protein kinase C inhibitor, thereby disrupting cell signaling mechanisms and inducing apoptosis.

The clinical feature that distinguishes hypericin from PUV A therapy is its lack of mutagenic activity in contrast to the mutagenic properties of psoralen and UV light. In addition, hypericin is maximally activated at 590nm, which is in the visible frequency of white light, rather than by long-wave UV -A which requires specialized lamps. Overall the data from this Phase II clinical trial support the contention that topical hypericin treatment combined with visible light is promising as a safer alternative to the standard PUV A therapy for the treatment of mild to moderate MF and psoriasis.

The amount of photosensitizer according to the present invention liquid formulation, preferably photosensitizers that exhibit cytotoxicity effect when exposed to light in the visible spectrum, is approximately 0.001% to 5%, preferably 0.001% to 1%, more preferably 0.02% to 0.5%. The typical amount of the photosensitizer applied to the skin is typically 0.0001 mg/cm² to 0.5 mg/cm², preferably 0.0001 mg/cm² to 0.1 mg/cm², more preferably 0.0001 mg/cm² to 0.05 mg/cm².

Particularly, the study unexpectedly discovers that the liquid hypericin formulation is superior compare to hypericin in hydrophilic ointment preparation. It is believed that solvent system used in the liquid hypericin formulation facilitates the permeation of hypericin through the psoriatic lesions. In the exemplary liquid formulation, DMSO and water solvent system is used. Preferably, 10-90% of DMSO is used with this liquid formulation, more preferably, 25-75% DMSO is used, and even more preferably, 40-60% DMSO is used.

Similar solvents that promote permeability of psoriatic lesions are also suitable for the present invention, which may include water, protic solvents, polar aprotic solvents, and polar aromatic solvents, and combinations thereof.

Any molecular solvent which contains dissociable H+, such is called a protic solvent. The molecules of such solvents can donate an H+ (proton). Conversely, aprotic solvents cannot donate hydrogen. Suitable protic solvents may include water, polyethylene glycols, propylene glycol, polyvinylpyrrolidone, methoxypropylene glycol, glycerol and glycofurol.

Polar aprotic solvents are solvents that share ion dissolving power with protic solvents but lack an acidic hydrogen. These solvents generally have high dielectric constants and high polarity. Polar aprotic solvents may include N-methylpyrrolidone, Nmethyl-2-pyrrolidone dimethylfonnamide, dimethylacetamide, dimethylformamide, Nmethyl-morpholine, y-butyrolactone, acetonitrile, hexamethylphosphorotriamide, and hexamethylphosphoramide.

Polar aromatic solvents may include haloaromatics, benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol;-2,2-dimethyl-3-(3-methylphenyl)propanol; I, 1-dimelhyl-2-phenylethyl alcohol; 1, 1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethano, benzyl acetate; benzyl propionate, benzyl isobutyrate; benzyl isovalerate, 2-phenylethyl acetate; 2- phenylethyl propionate, 2-phenylethyl isobutyrate, 2-phenylethyl isovalerate, 1-phenylethyl acetate, alpha-trichloromethylbenzyl acetate, alpha,alpha-dimethylphenylethyl acetate, alpha,alpha-dimethylphenylethyl butyrate; cinnamyl acetate, 2-phenoxyethyl isobutyrate, 4-methoxybenzyl acetate, araliphatic ethers such as, for example, 2-phenylethyl methyl ether, 2-phenylethyl isoamyl ether, 2-phenylethyl-1-ethoxyethyl ether, phenylacetaldehyde dimethyl acetal, phenylacetaldehyde dietheyl acetal, hydratropaldehyde dimethyl acetal, phcnylacetaldehyde glycerol acetal, 2,4,6-trimethyl-4-phenyl-1,3-dioxane, 4,4a,5,9b-tetrahydroindeno[1,2-d]-m-dioxin, and 4,4a,5,9b-tetrahydro-2,4-dimethylindeno[1,2-d]-m-dioxin.

Additionally, surfactants may be also used to facilitate skin permeability. Surfactants may include alkyl aryl sulfonates, alkyl sulfates, sulfonated amides and amines, sulfated and sulfonated esters and ethers, alkyl sulfonates, polyethoxlyated esters, mono- and diglycerides, diacetyl tartaric esters of monoglycerides, polyglycerol esters, sorbitan esters and ethoxylates, lactylated esters, phospholipids, polyoxyethylene sorbitan esters, propylene glycol esters, and sucrose esters.

Other skin permeation enhancers may also be used with the present invention formulation. Other skin permeation enhancers include decylmethylsulfoxide, 1-decyl methyl sulfoxide, 1-decyl methyl sulfoxide analogs, diethylene glycol monoethyl ether, polyethylene glycol monolaurate, glycerol monolaurate, propylene glycol monolaurate, ethanol, eucalyptol, lecithin, the 1-substituted azacycloheptan-2-ones, particularly 1-n-dodecylcyclazacycloheptan-2-one (available under the trademark Azone® from Nelson Research & Development Co., Irvine, CA), "cell envelope disordering compounds" such as methyl laurate or oleic acid in combination with N-(hydroxyethyl) pyrrolidone, C3-C4 diols, or a binary system of oleic acid, oleins or oleyl alcohol in combination with a lower alcohol.

It is also discovered that such liquid is used to increase the efficacy of over psoriasis treatment, for example, without limitation, psoralen, glucocorticoids, calcineuron inhibitors (e.g., tacrilimus), and histone deacetylase inhibitors.

It is also discovered that a method of photodynamic therapy using light in the visible spectrum can be effectively used to for the treatment of a skin condition associated with leukocytes accumulation in the skin. These conditions may include Cutaneous T cell lymphoma and psoriasis. Comparing to photodynamic therapy using UV light, using visible light is much safer, since it eliminates the harmful mutagenic and carcinogenic effect of UV light. One ordinary skilled in the art would select one or more appropriate photosensitizers that exhibit cytotoxicity effect when exposed to visible light of a particular wavelength, apply the selected photosensitizer to a skin lesion of the patient that is afflicted with the skin condition, and expose the treated skin area with the appropriate spectrum of visible light. Hypericin is shown to be safe and effective for the treatment of CTCL and psoriasis according to this method. Photosensitizers suitable for this particular method are preferably skin permeable, or may be made skin permeable with solvent or skin permeation enhancers. It is also preferable that the photosensitizers would produce singular oxygen when exposed to light in the visible spectrum. Other photosensitizers may be used according to this method. As an example without limitation, aminolevulinic acid, may be applied topically in 1 0 to 20% concentrations and exposed to light at 600-700 nm.

The above detailed liquid formations and methods of treatment can be adapted to the treatment of warts. Particularly, hypericin photodynamic therapy may be used. In this particular example, hypericin is, *in vitro,* established to have a light-activated viricidal activity. Light activation of hypericin (at concentrations of 1 ∼ 20 J.tg) for 15 - 30 minutes inactivates infectious titers (5 - 6 log₁₀) of HIV and of bovine diarrhea virus (BVDV).

As used herein, the term "hypericin and hypericin derivative" means hypericin, protohypericin, pseudohypericin, helianthrone, or a combination thereof. It is clear to one of skill in the art that numerous insignificant modifications may be made to the chemical structure of hypericin, protohypericin, pseudohypericin, or helianthrone and that many such modifications will not significantly alter the biological activity of the molecule. Hence, hypericin, protohypericin, pseudohypericin, and helianthrone molecules which have been insignificantly modified, such that the biological activity is not significantly altered, are included within the definition of an appropriate hypericin derivative. One of skill in the art will appreciate that they may use the methods taught herein to distinguish modified hypericin, protohypericin, pseudohypericin, or helianthrone molecules having insignificantly altered biological activity from those having significanlly altered biological activity.

As used herein, the term "visible light" and "light in the visible spectn1m" means the portion of the electromagnetic spectrum that is visible to or can be detected by the human eye. Electromagnetic radiation in this range of wavelengths is called visible light or simply light. A typical human eye will respond to wavelengths from about 390 to 750 nm. Typically, violet light is between about 380-450 nm, blue light is between about 450-495 nm, green light is between about 495-570 nm, yellow light is between about 570-590 nm, orange light is between about 590-620 run, and red light is between about 620-750 nm.

## Claims

1. A liquid formulation for use in the topical treatment of a skin condition selected from the group consisting of cutaneous T cell lymphoma (CTCL) and psoriasis, comprising:
a photosensitizer that is skin permeable, wherein the photosensitizer is hypericin or a hypericin derivative; and
an organic solvent selected from polar aprotic solvents.

2. The liquid formulation for use of claim 1 comprising
the photosensitizer in the amount of 0.001% to 1%, wherein the photosensitizer exhibits cytotoxicity when exposed to light in the visible spectrum; and
the organic solvent in the amount of 25% to 75%.

3. The liquid formulation for use of claim 1, wherein the organic solvent is dimethyl sulfoxide (DMSO).

4. The liquid formulation for use of claim 1, wherein the treatment of a skin condition comprises the steps of:
applying topically the liquid formulation to a skin lesion of a patient afflicted with the skin condition; and
exposing the skin lesion to a light source.

5. The liquid formulation for use of claim 4, wherein the light source is a visible light source.

6. The liquid formulation for use of claim 4, wherein the light source is a UV-A light source.

7. The liquid formulation for use of claim 4, wherein the light source is at an energy level of 4 to 20 J/cm².

8. The liquid formulation for use of claim 4, wherein the light source emits light having a wavelength between 500 nm to 650 nm, a wavelength between 390 nm to 750 nm, a wavelength between 380 nm to 750 nm, a wavelength between 495 nm to 570 nm, a wavelength between 450 nm to 495 nm, a wavelength between 570 nm to 590 nm, a wavelength between 590 nm to 620 nm, or a wavelength between 620 nm to 750 nm.

9. The liquid formulation for use of claim 1, wherein the skin condition is selected from mycosis fungoides (MF) or Sezary syndrome.

## Patentansprüche

1. Flüssige Formulierung zur Verwendung bei der topischen Behandlung eines Hautleidens, ausgewählt aus der Gruppe bestehend aus kutanem T-Zell-Lymphom (CTCL) und Psoriasis, umfassend:
einen Photosensibilisator, der für die Haut durchlässig ist, wobei der Photosensibilisator Hypericin oder ein Hypericin-Derivat ist; und
ein organisches Lösungsmittel, ausgewählt aus polaren aprotischen Lösungsmitteln.

2. Flüssige Formulierung zur Verwendung nach Anspruch 1, umfassend den Photosensibilisator in einer Menge von 0,001 % bis 1 %, wobei der Photosensibilisator Zytotoxizität zeigt, wenn er Licht im sichtbaren Spektrum ausgesetzt wird; und
das organische Lösungsmittel in einer Menge von 25% bis 75%.

3. Flüssige Formulierung zur Verwendung nach Anspruch 1, wobei das organische Lösungsmittel Dimethylsulfoxid (DMSO) ist.

4. Flüssige Formulierung zur Verwendung nach Anspruch 1, wobei die Behandlung eines Hautleidens die folgenden Schritte umfasst:
das Auftragen der flüssigen Formulierung auf eine Hautläsion eines von dem Hautleiden betroffenen Patienten; und
die Hautläsion einer Lichtquelle aussetzen.

5. Flüssige Formulierung zur Verwendung nach Anspruch 4, wobei die Lichtquelle eine Quelle von sichtbarem Licht ist.

6. Flüssige Formulierung zur Verwendung nach Anspruch 4, wobei die Lichtquelle eine UV-A-Lichtquelle ist.

7. Flüssige Formulierung zur Verwendung nach Anspruch 4, wobei die Lichtquelle ein Energieniveau von 4 bis 20 J/cm2 hat.

8. Flüssige Formulierung zur Verwendung nach Anspruch 4, wobei die Lichtquelle Licht mit einer Wellenlänge zwischen 500 nm und 650 nm, einer Wellenlänge zwischen 390 nm und 750 nm, einer Wellenlänge zwischen 380 nm und 750 nm, einer Wellenlänge zwischen 495 nm und 570 nm, einer Wellenlänge zwischen 450 nm und 495 nm, einer Wellenlänge zwischen 570 nm und 590 nm, einer Wellenlänge zwischen 590 nm und 620 nm oder einer Wellenlänge zwischen 620 nm und 750 nm emittiert.

9. Flüssige Formulierung zur Verwendung nach Anspruch 1, wobei das Hautleiden ausgewählt ist aus Mycosis fungoides (MF) oder dem Sezary-Syndrom.

## Revendications

1. Formulation liquide destinée à être utilisée dans le traitement topique d'une affection cutanée choisie dans le groupe constitué par le lymphome cutané à cellules T (CTCL) et le psoriasis, comprenant :
un photosensibilisant qui est perméable à la peau, dans laquelle le photosensibilisant est l'hypéricine ou un dérivé d'hypéricine ; et
un solvant organique choisi parmi des solvants polaires aprotiques.

2. Formulation liquide à utiliser selon la revendication 1, comprenant
le photosensibilisant en la quantité de 0,001 % à 1 %, dans laquelle le photosensibilisant présente une cytotoxicité lorsqu'il est exposé à de la lumière dans le spectre visible ; et
le solvant organique en la quantité de 25 % à 75 %.

3. Formulation liquide à utiliser selon la revendication 1, dans laquelle le solvant organique est le diméthylsulfoxyde (DMSO).

4. Formulation liquide à utiliser selon la revendication 1, dans laquelle le traitement d'une affection cutanée comprend les étapes consistant à :
appliquer de manière topique la formulation liquide sur une lésion cutanée d'un patient atteint de l'affection cutanée ; et
exposer la lésion cutanée à une source de lumière.

5. Formulation liquide à utiliser selon la revendication 4, dans laquelle la source de lumière est une source de lumière visible.

6. Formulation liquide à utiliser selon la revendication 4, dans laquelle la source de lumière est une source de lumière UV-A.

7. Formulation liquide à utiliser selon la revendication 4, dans laquelle la source de lumière est à un niveau d'énergie de 4 à 20 J/cm².

8. Formulation liquide à utiliser selon la revendication 4, dans laquelle la source de lumière émet de la lumière ayant une longueur d'onde entre 500 nm et 650 nm, une longueur d'onde entre 390 nm et 750 nm, une longueur d'onde entre 380 nm et 750 nm, une longueur d'onde entre 495 nm et 570 nm, une longueur d'onde entre 450 nm et 495 nm, une longueur d'onde entre 570 nm et 590 nm, une longueur d'onde entre 590 nm et 620 nm ou une longueur d'onde entre 620 nm et 750 nm.

9. Formulation liquide à utiliser selon la revendication 1, dans laquelle l'affection cutanée est choisie parmi la mycose fongoïde (MF) ou le syndrome de Sézary.
